# EUROPEAN PATENT APPLICATION

(11) **EP 4 544 995 A1**
(43) Date of publication of application: **30.04.2025**
(21) Application number: 23213644.0
(22) Date of filing: 01.12.2023
(51) Int. Cl.: A61B 5/024, A61B 5/344, A61B 5/00

(54) **SYSTEMS AND METHODS FOR ELECTRODE POSITION DETERMINATION FOR FETAL MONITORING SYSTEM**

(30) Priority: 26.10.2023 US 202363545767 P
(71) Applicant: Koninklijke Philips N.V., 5656 AG Eindhoven (NL)
(72) Inventor: HAMELMANN, Paul Christoph, Eindhoven (NL); HIDALGO ARAYA, Marco, Eindhoven (NL); RABOTTI, Chiara, 5656AG Eindhoven (NL)
(74) Representative: Philips Intellectual Property & Standards

(57) **Abstract**

A fetal monitoring system including a plurality of measurement electrodes, a reference electrode, and a processor is provided. The plurality of measurement electrodes and the reference electrode are configured to be arranged on an abdomen of a user. The processor is electrically coupled to the plurality of measurement electrodes and the reference electrode. The processor is configured to generate a plurality of electrophysiological signals. The plurality of electrophysiological signals correspond to the plurality of measurement electrodes and the reference electrode. The processor is further configured to extract one or more features from the plurality of electrophysiological signals. The processor is further configured to determine electrode position data of each of the plurality of measurement electrodes based on the one or more features.

## Description

### FIELD OF THE INVENTION

The present disclosure is generally directed to a fetal monitoring system and, more particularly, to systems and methods for determining positions of electrodes of the fetal monitoring system.

### BACKGROUND OF THE INVENTION

There is a strong trend towards remote fetal monitoring solutions using electrophysiology based-cardiotocography (eCTG). An essential part of these solutions is a fetal monitoring device which can be easily self-applied by a pregnant mother in the home setting. For high-quality measurements, it is essential that the electrodes of the device are placed in the correct location on the maternal abdomen. However, in some instances, the fetal monitoring device may be placed incorrectly despite clear instructions being provided with the device. This incorrect placement is exacerbated by the lack of professional oversight of the placement of the fetal monitoring device during remote use.

### SUMMARY OF THE INVENTION

The present disclosure is generally directed to systems and methods for determining positions of electrodes of the fetal monitoring system. The fetal monitoring system may be embodied as a plurality of measurement electrodes, a ground electrode, a reference electrode, and a controller. The controller, including a processor, a memory, and a transceiver, is electrically coupled to the measurement electrodes, the ground electrode, and the reference electrode. The various electrodes are arranged on the abdomen of the user. The controller processes electrical signals from the various electrodes to generate a plurality of electrophysiological signals. Features which may be indicative of electrode position are then extracted from the electrophysiological signals. These features are then analyzed to determine electrode position data for the measurement electrodes.

In some examples, the electrode position data is determined based on maternal heart activity. In particular, the major direction of electrical activity of an adult heart (referred to as an electrical heart axis) is fixed and pointing in a downward-left direction. The position of a measurement electrode relative to the electrical heart axis is reflected in electrocardiogram (ECG) waveforms corresponding to the measurement electrode and a reference electrode. More specifically, the direction of R-peak components of the ECG waveforms may be indicative of the position of the measurement electrode.

In some examples, the electrode position data may be used to provide feedback to the user regarding the positioning of the electrodes. For example, the controller may process the electrode position data to generate user feedback data. The user feedback data may include notifications regarding the current position of the measurement electrodes and instructions for repositioning the measurement electrodes, if necessary.

In some examples, the electrode position data is used to determine fetal heart rate. For example, the electrode position data may be used to determine a subset of the electrophysiological signals most indicative of fetal heart rate. The controller may then use this subset of electrophysiological signals to determine fetal heart rate.

In some examples, aspects of the electrode position data is used to generate uterine activity data. For example, the electrode position data representative of the spatial distribution of the measurement electrodes may be used with the electrophysiological signals to evaluate uterine activity.

In some examples, the electrode position data may be tracked over a time period and used to standardize an electrophysiological measurement set generated over the time period. This standardization may be particularly useful if the measurement electrodes have been moved by the user during the time period.

Generally, in one aspect, a fetal monitoring system is provided. The fetal monitoring system includes a plurality of measurement electrodes. The plurality of measurement electrodes is configured to be arranged on an abdomen of a user.

The fetal monitoring system further includes a reference electrode. The reference electrode is also configured to be arranged on the abdomen of the user.

The fetal monitoring system further includes a processor. The processor is electrically coupled to the plurality of measurement electrodes and the reference electrode.

The processor is configured to generate a plurality of electrophysiological signals. The plurality of electrophysiological signals correspond to the plurality of measurement electrodes and the reference electrode.

The processor is further configured to extract one or more features from the plurality of electrophysiological signals.

The processor is further configured to determine electrode position data of each of the plurality of measurement electrodes based on the one or more features.

According to an example, the processor is further configured to generate user feedback data based on the electrode position data. Further to this example, the fetal monitoring system may further include a display screen configured to show the user feedback data. Additionally, the fetal monitoring system may further include a transceiver. The transceiver may be configured to transmit the user feedback data to an external device.

According to an example, the one or more features may include electrocardiogram (ECG) waveform data. The ECG waveform data may at least partially correspond to maternal heart activity of the user. The electrode position data may be determined based on R-peak data of the ECG waveform data.

According to an example, the processor is further configured to generate fetal heart rate data. The fetal heart rate data is generated based on a subset of the plurality of electrophysiological signals. The subset is selected according to the electrode position data.

According to an example, the processor is further configured to generate uterine activity data. The uterine activity data is generated based on one or more of the plurality of electrophysiological signals and the electrode position data of one or more of the plurality of measurement electrodes.

According to an example, the processor is further configured to collect an electrode position data set of the electrode position data determined over a time period. The processor is further configured to calibrate an electrophysiological measurement set captured over the time period based on the electrode position data set.

According to an example, the fetal monitoring system further includes at least one ground electrode.

Generally, in another aspect, a method for fetal monitoring is provided. The method includes generating, via a processor, a plurality of electrophysiological signals corresponding to a plurality of measurement electrodes and a reference electrode. The plurality of measurement electrodes and the reference electrode are arranged on an abdomen of a user.

The method further includes extracting, via the processor, one or more features from the plurality of electrophysiological signals.

The method further includes determining, via the processor, electrode position data of each of the plurality of measurement electrodes based on the one or more features.

According to an example, the method further includes generating, via the processor, user feedback data based on the electrode position data. The method further includes showing, via a display screen, the user feedback data.

According to an example, the method further includes generating, via the processor, fetal heart rate data based on a subset of the plurality of electrophysiological signals selected according to the electrode position data.

In various implementations, a processor or controller may be associated with one or more storage media (generically referred to herein as "memory," e.g., volatile and non-volatile computer memory such as RAM, PROM, EPROM, EEPROM, floppy disks, compact disks, optical disks, magnetic tape, SSD, etc.). In some implementations, the storage media may be encoded with one or more programs that, when executed on one or more processors and/or controllers, perform at least some of the functions discussed herein. Various storage media may be fixed within a processor or controller or may be transportable, such that the one or more programs stored thereon can be loaded into a processor or controller so as to implement various aspects as discussed herein. The terms "program" or "computer program" are used herein in a generic sense to refer to any type of computer code (e.g., software or microcode) that can be employed to program one or more processors or controllers.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the various embodiments.
Fig. 1 is an illustration of a system for fetal monitoring arranged on a user, in accordance with an example.
Fig. 2 is a further illustration of the system for fetal monitoring of Fig. 1.
Fig. 3 illustrates a series of electrocardiogram signals generated by the system for fetal monitoring, in accordance with an example.
Fig. 4 is a functional block diagram of a system for fetal monitoring, in accordance with an example.
Fig. 5 is a functional block diagram of a system for fetal monitoring configured to determine electrode position, in accordance with an example.
Fig. 6 is a functional block diagram of a system for fetal monitoring configured to provide user feedback regarding electrode position, in accordance with an example.
Fig. 7 is a functional block diagram of a system for fetal monitoring configured to determine fetal heart rate, in accordance with an example.
Fig. 8 is a functional block diagram of a system for fetal monitoring configured to capture uterine activity, in accordance with an example.
Fig. 9 is a functional block diagram of a system for fetal monitoring configured to standardize electrophysiological measurements captured over a time period, in accordance with an example.
Fig. 10 is a schematic diagram of a controller of a system for fetal monitoring, in accordance with an example.
Fig. 11 is a flow chart of a method for fetal monitoring, in accordance with an example.

### DETAILED DESCRIPTION OF EMBODIMENTS

The present disclosure is generally directed to systems and methods for determining positions of electrodes of the fetal monitoring system. The fetal monitoring system may be embodied as a plurality of measurement electrodes, a ground electrode, a reference electrode, and a controller. The controller, including a processor, a memory, and a transceiver, is electrically coupled to the measurement electrodes, the ground electrode, and the reference electrode. The various electrodes are arranged on the abdomen of the user. The controller processes electrical signals from the various electrodes to generate a plurality of electrophysiological signals. Features which may be indicative of electrode position are then extracted from the electrophysiological signals. These features are then analyzed to determine electrode position data for the measurement electrodes.

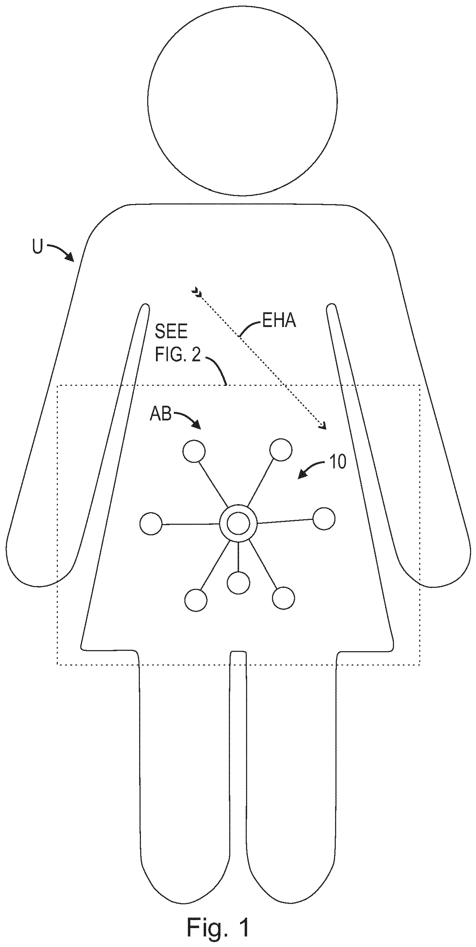

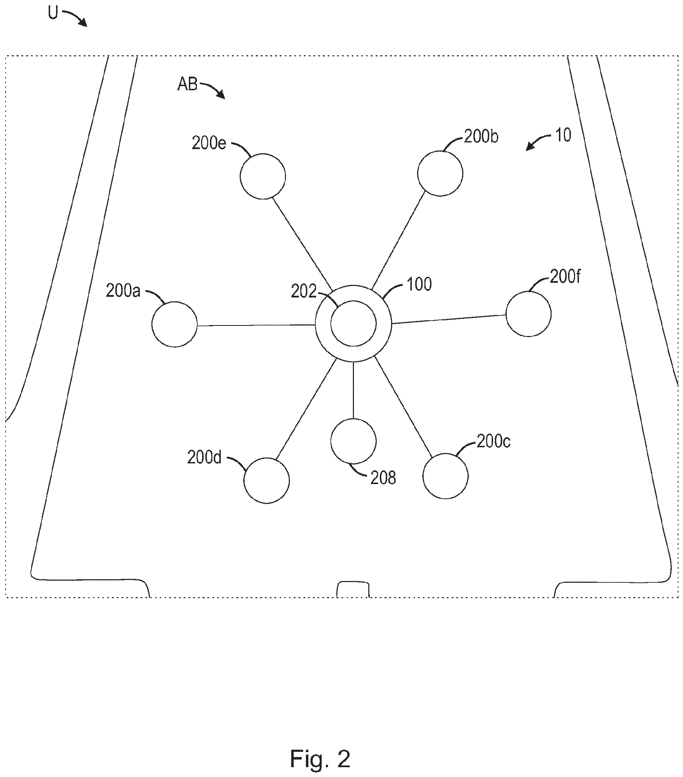

Turning now to the figures, Fig. 1 illustrates a non-limiting example of a fetal monitoring system 10 according to various embodiments of the present disclosure, arranged on an abdomen AB of a user U, while Fig. 2 illustrates a portion of Fig. 1. As shown in Figs. 1 and 2, the non-limiting example of the fetal monitoring system 10 includes a controller 100, six measurement electrodes 200a-f, a reference electrode 202, and a ground electrode 208. The measurement electrodes 200a-f, the reference electrode 202, and the ground electrode 208 are each arranged to be in contact with the abdomen AB of the user U. While the fetal monitoring system 10 illustrates six measurement electrodes 200a-f, any practical number of measurement electrodes 200a-f may be used. In some examples, the fetal monitoring system 10 may have as few as one measurement electrode 200, while in some other examples, the fetal monitoring system 10 may have more than six. These measurement electrodes 200a-f may be arranged in any practical configuration or pattern on the user U. While the six measurement electrodes of 200a-f shown in Fig. 1 are positioned to contact the abdomen AB, the system 10 may also include other measurement electrodes 200 arranged in other positions on the user U. For example, some electrodes 200 may be arranged on the chest area, back area, or arm area of the user U.

In the non-limiting example of Fig. 1, the measurement electrodes 200a-f and the ground electrode 208 are electrically coupled to the controller 100 via a series of lead wires. Further, the reference electrode 202 is embedded within the same housing as the controller 100. However, in other examples, the reference electrode 202 and the controller 100 may be arranged as discrete components electrically coupled via a lead wire. Further to these alternative examples, the controller 100 may be arranged off of the body of the user U, such as part of a patient monitoring station or other external monitoring device.

The controller 100 uses the aforementioned measurement electrodes 200a-f, reference electrode 202, and ground electrode 208 to generate a series of electrophysiological signals 102a-f. The electrophysiological signals 102a-f represent electrical activity of a maternal heart, a fetal heart, a uterus, and other muscles (such as skeletal muscles), as well as electrical noise. Preferably the fetal monitoring system 10 is arranged centrally on the abdomen AB of the user U to increase the chance that the fetal heart is spatially within the measurement electrodes 200a-f and the uterus is proximate to the measurement electrodes 200a-f. This arrangement should increase signal amplitude of fetal electrocardiogram signals and uterine electrophysiological signals, thereby allowing for easier detection of fetal heart rate and uterine activity.

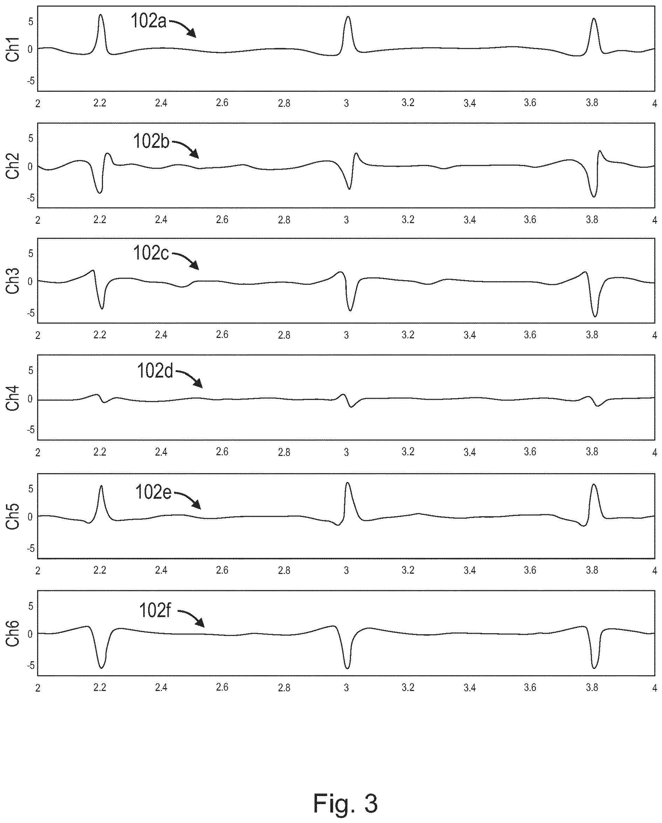

A series of example electrophysiological signals 102a-f are shown in Fig. 3. Each of the electrophysiological signals 102a-f are determined based on electrophysiological-voltage differences between one of the measurement electrodes 200a-f and the reference electrode 202. For example, the first electrophysiological signal 102a (channel 1 in Fig. 3) represents the voltage difference between a first measurement electrode 200a and the reference electrode 202, the second electrophysiological signal 102b (channel 2 in Fig. 3) represents the voltage difference between a second measurement electrode 200b and the reference electrode 202, and so on. These electrophysiological signals 102a-f may be determined relative to ground signal 210 corresponding to the ground electrode 208.

The electrophysiological signals 102a-f may be processed to derive various types of features 104 corresponding to the position of the measurement electrodes 200a-f on the abdomen AB of the user U. For example, Fig. 1 illustrates an electrical heart axis EHA. The electrical heart axis EHA illustrates the fixed nature of the primary direction of overall electrical activity of the adult heart being in a downward-left direction. The amplitude and the shape of the electrophysiological signals 102a-f reflect the orientation of the measuring electrode couple (the measurement electrode 200a-f to the reference electrode 202) relative to the electrical heart axis EHA. In the example of Fig. 3, the direction of the R-peak of each of the electrophysiological signals 102a-f depends on the orientation of the electrode couple to the electrical heart axis EHA. As shown in Fig. 3, the first, fourth, and fifth electrophysiological signals 102a, 102d, 102e include R-peaks pointing upwards, indicative of the first, fourth, and fifth measure electrodes 202a, 202d, 202e being arranged on the right side of the abdomen AB of the user U. Further, the second, third, and sixth electrophysiological signals 102b, 102c, 102f include R-peaks pointing downwards, indicative of the second, third, and sixth measurement electrodes 202b, 202c, 202f being arranged on the left side of the abdomen AB of the user U. In some examples, standard R-peak detection algorithms, such as the Pan-Tompkins algorithm, may be used to detect the R-peak direction of each of the electrophysiological signals 102a-f.

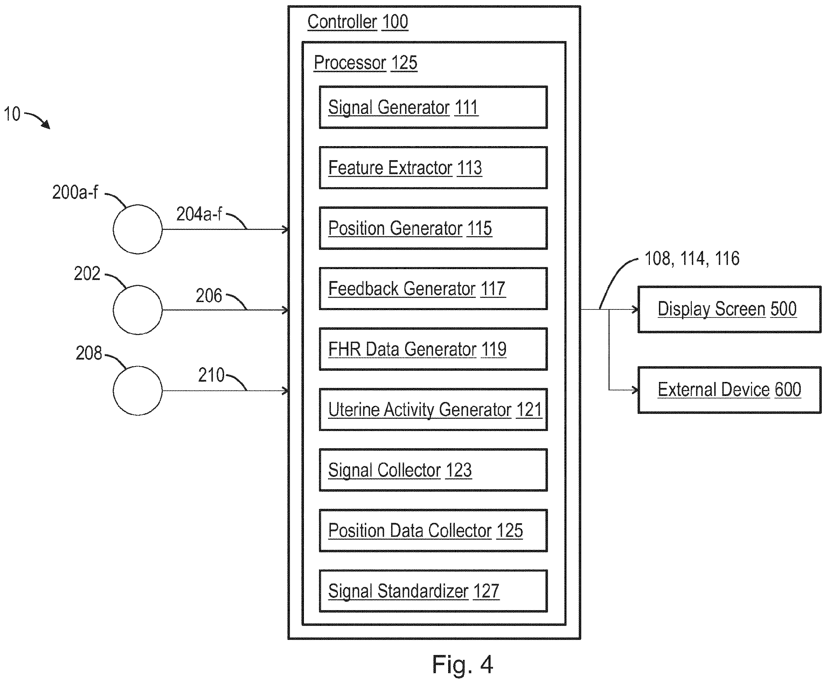

Fig. 4 illustrates a functional block diagram of a non-limiting example of a system 10 for fetal monitoring. Generally, the system 10 analyzes data collected via the plurality of measurement electrodes 200a-f, the reference electrode 202, and the ground electrode 208 to provide a user with user feedback 108 regarding the position of the measurement electrodes 200a-f. In addition to user feedback 108, the fetal monitoring system 108 may also utilize information regarding the position of the measurement electrodes 200a-f to determine fetal heart rate data 114 and/or uterine activity data 116. The measurement electrodes 200a-f provide measurement signals 204a-f to the controller 100. The reference electrode 202 provides reference signal 206 to the controller 100. The ground electrode 208 provides a ground signal 210 to the controller 100. The user feedback 108, fetal heart rate data 114, and/or the uterine activity data 116 may be visualized on a display screen 500 or transmitted to an external device 600.

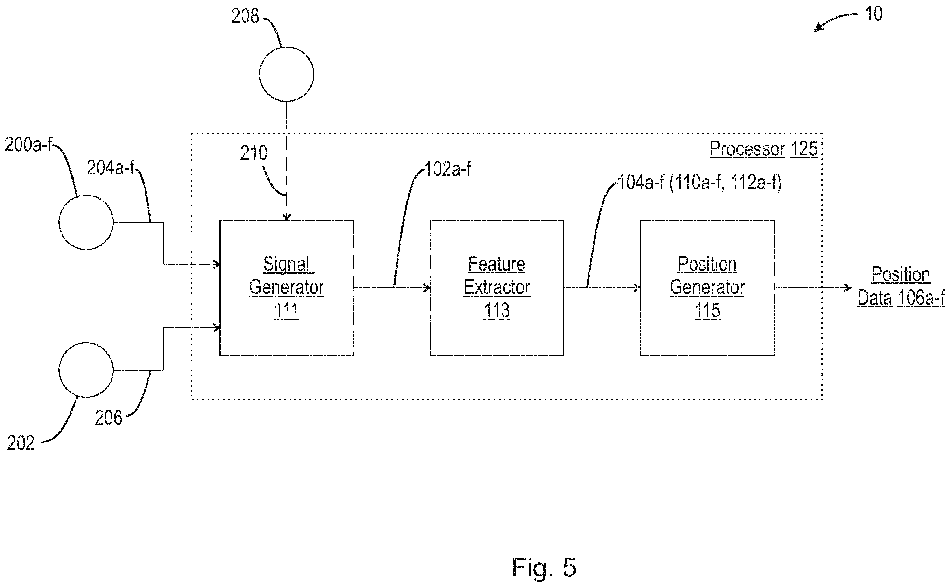

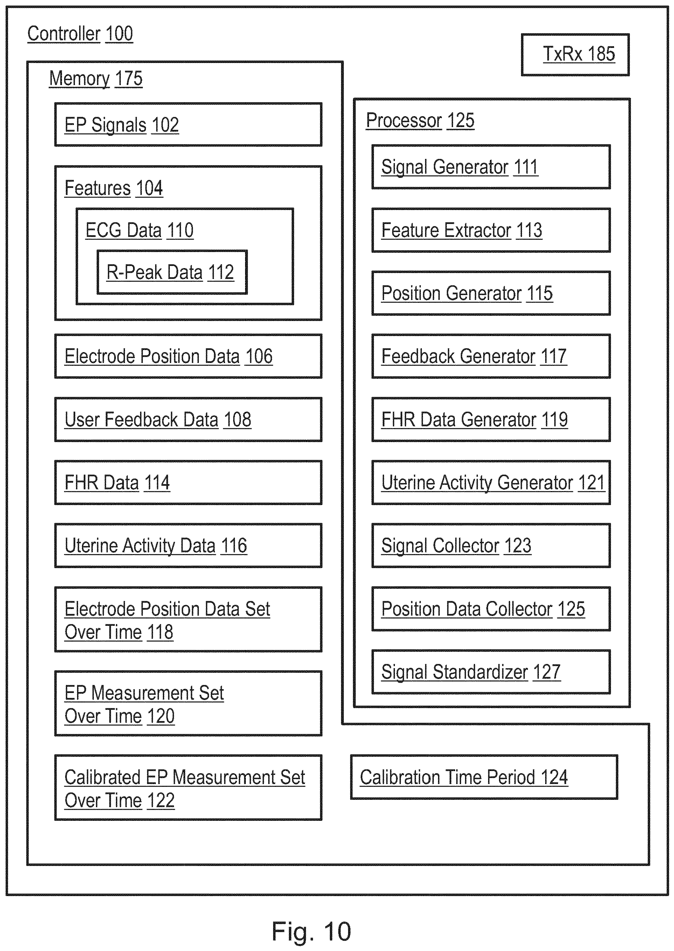

With continued reference to Fig. 4, the system 10 includes a controller 100. The controller 100, shown in more detail in Fig. 10, includes a processor 125, a memory 175, and a transceiver 185. The processor 125 is configured to execute a variety of modules to determine various aspects of the system 10. In the example of Fig. 4, the processor 125 is configured to execute an electrophysiology signal generator 111, a feature extractor 113, an electrode position generator 115, a user feedback generator 117, a fetal heart rate data generator 119, a uterine activity generator 121, an electrophysiology signal collector 123, an electrode position data collector 125, and a signal standardizer 127. These modules are described in further detail with reference to Figs. 5-9. The memory 175 is configured to store data received by the controller 100 via wired or wireless connection. The memory 175 is further configured to store data generated by the processor 125.

The transceiver 185 is configured to wirelessly transmit data to or wirelessly receive data from various aspects of the system 10. The transceiver 185 may also enable wireless communication with components arranged externally to the system 10. The transceiver 185 may wirelessly transmit data to a display screen 500, such as a dashboard displaying information such as (but not necessarily limited to) user feedback 108, fetal heart rate data 114, and uterine activity data 116. The transceiver 185 may also wirelessly transmit the aforementioned data to an external device 600, such as a mobile phone, tablet computer, or central monitoring station.

Fig. 5 is another exemplary functional block diagram of the system 10 for fetal monitoring. In particular, Fig. 5 illustrates the flow of data between the various modules and programs executed by the processor 125 of the controller 100 to determine the position of the measurement electrodes 200a-f based on the electrophysiological signals 102a-f corresponding to the measurement electrodes 200a-f, the reference electrode 202, and the ground electrode 208.

The measurement electrodes 200a-f, the reference electrode 202, and the ground electrode 208 are electrically coupled to the electrophysiology signal generator 111. As shown in Figs. 1 and 2, the measurement electrodes 200a-f, the reference electrode 202, and the ground electrode 208 are arranged on the abdomen AB of the user U. The electrophysiology signal generator 111 processes signals received from the measurement electrodes 200a-f, the reference electrode 202, and the ground electrode 208 to generate an electrophysiology signal 102a-f corresponding to each measurement electrode 200a-f as shown in Fig. 3. As previously described, the electrophysiology signals 102a-f may correspond to electrical activity of the maternal heart, the fetal heart, the uterus, and other muscles, as well as electrical noise.

The electrophysiology signals 102a-f are then provided to a feature extractor 113. The feature extractor 113 derives one or more features 104a-f from the electrophysiology signals 102a-f indicative of the position of the measurement electrodes 200a-f. In one example, the features 104a-f include electrocardiogram (ECG) waveform data 110a-f, such as R-peak data 112a-f. In some examples, the R-peak data 112a-f may be determined by processing the electrophysiology signals 102a-f with the Pan-Tompkins algorithm.

The features 104a-f are then provided to an electrode position generator 115. The electrode position generator 115 analyzes the features 104a-f to determine electrode position data 106a-f for each measurement electrode 200a-f. In some examples, this determination may be based on the direction of the R-peaks of the electrophysiology signals 102a-f corresponding to the measurement electrodes 200a-f. The electrode position generator 115 may also take additional factors into account, such as the physical configuration of the fetal monitoring device 10.

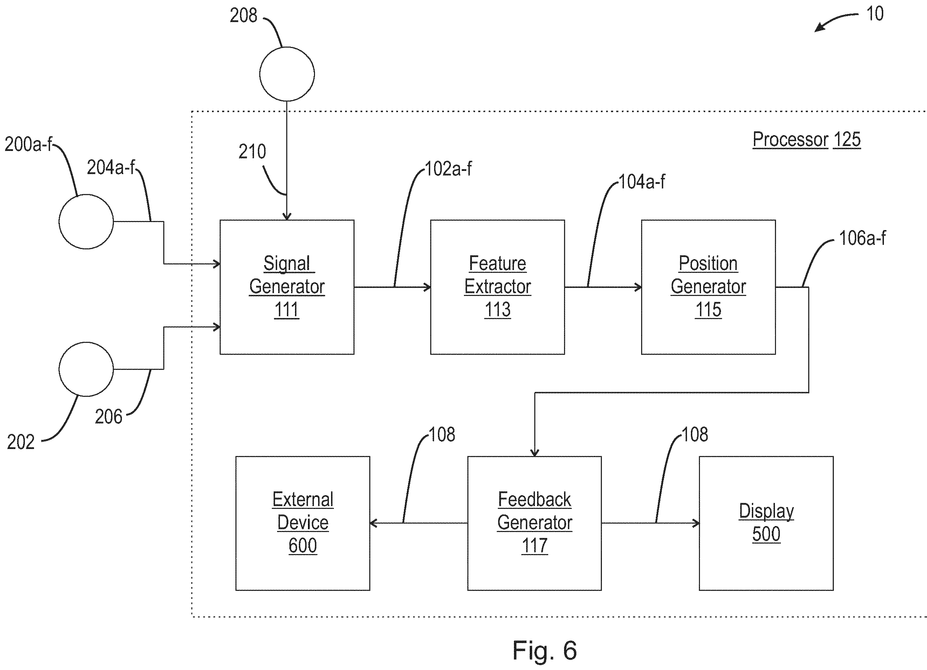

Fig. 6 is a variation of Fig. 5 illustrating an application of the electrode position data 106a-f generated in Fig. 5. In Fig. 6, the electrode position data 106a-f is further processed by a user feedback generator 117 to generate user feedback 108. The user feedback 108 is then provided to the user U via the display 500 or the external device 600.

The user feedback 108 may be embodied in a variety of forms. Minimally, the user feedback 108 should inform the user U that one or more aspects of the fetal monitoring device 10 (measurement electrodes 200, reference electrode 202, ground electrode 208) must be repositioned. In some examples, the user feedback 108 provides the user U with enough guidance to successfully reposition the out-of-position aspects of the fetal monitoring device 10.

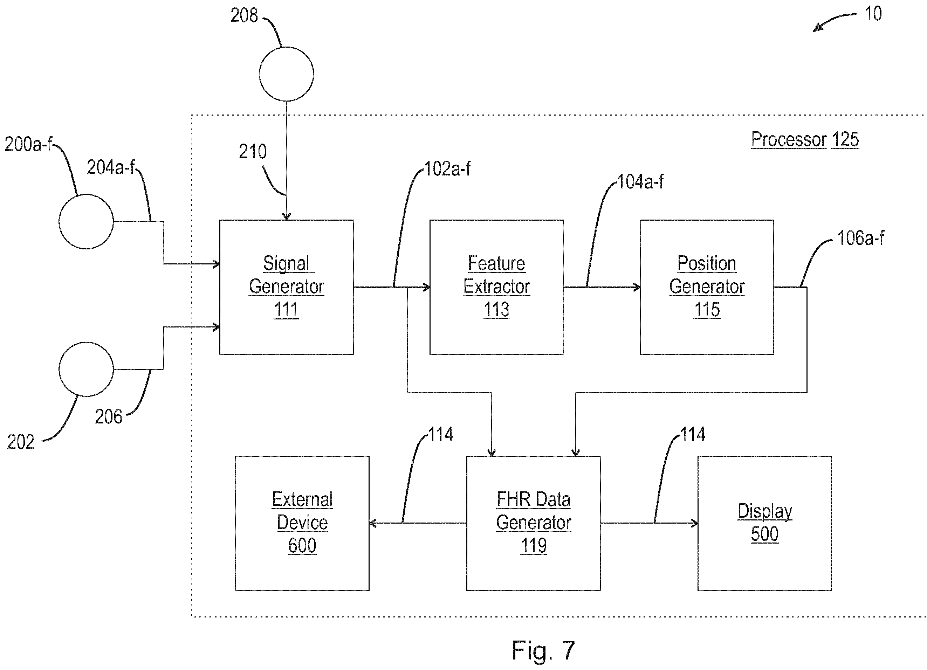

Fig. 7 is a variation of Figs. 5 and 6 illustrating a further application of the electrode position data 106a-f generated in Fig. 5. In Fig. 7, the electrode position data 106a-f is further processed by a fetal heart rate data generator 119 to optimize a fetal heart rate detection algorithm. In particular, the fetal heart rate generator 119 may use the electrode position data 106a-f to determine an optimized subset of the electrophysiological signals 102a-f to generate fetal heart rate data 114. In some examples, the optimized subset may include particular electrophysiological signals 102a-f in which fetal heart rate activity is more easily recognized. The optimized subset of electrophysiological signals 102a-f may change over time due to the fetus changing positions. The fetal heart rate data 114 may then be provided to the user via the display screen 500 or the external device 600.

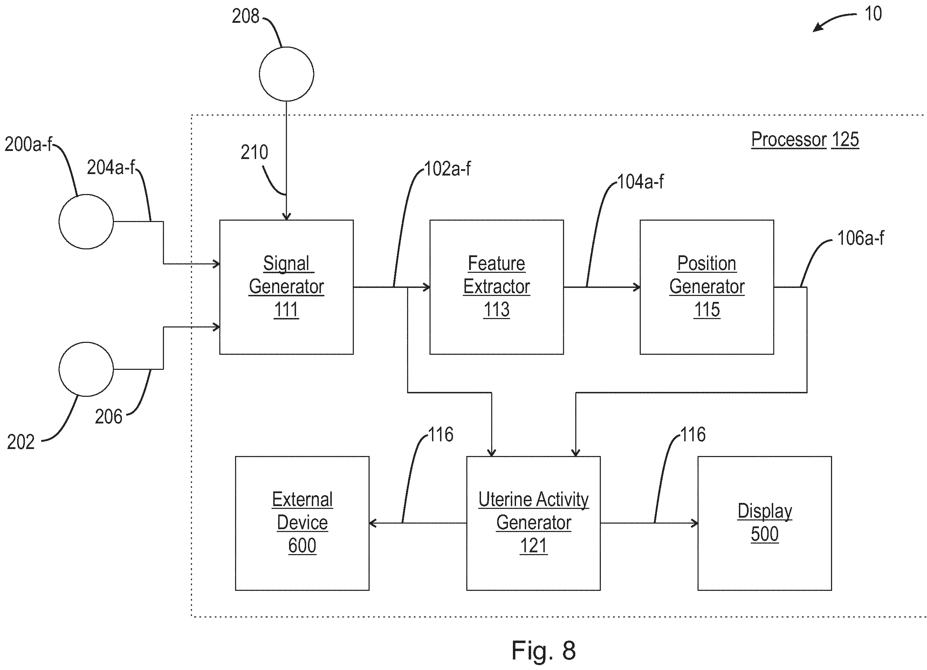

Fig. 8 is a variation of Figs. 5-7 illustrating a further application of the electrode position data 106a-f generated in Fig. 5. In Fig. 8, the electrode position data 106a-f is further processed by a uterine activity generator 121 to generate uterine activity data 116 during pregnancy and labor. The uterine activity generator 121 is able to use to augment the electrophysiological signals 102a-f with the electrode position data 102a-f to evaluate the spatial distribution of electrophysiological activity corresponding to uterine activity. The uterine activity data 116 may then be provided to the user via the display screen 500 or the external device 600.

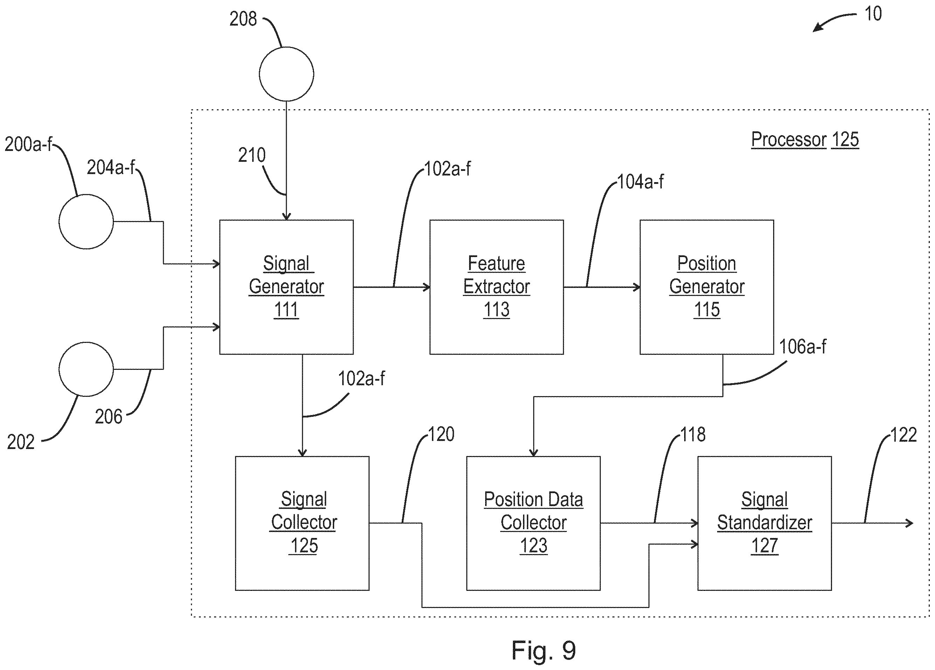

Fig. 9 is a further variation of Figs. 5-8 illustrating a further application of the electrode position data 106a-f generated in Fig. 5. In Fig. 9, the electrode position data 106a-f is tracked over time to standardize an electrophysiological measurement set 120. Standardization of the electrophysiological measurement set 120 may account for movement of aspects of the fetal monitoring system 10, such as one or more of the measurement electrodes 200a-f, over time. The position data collector 123 receives the electrode position data 106a-f generated over a calibration time period 124 and compiles the electrode position data 106a-f as an electrode position data set 118. Similarly, the electrophysiological signal collector 125 compiles the electrophysiological signals 102a-f generated over the calibration time period, and compiles the electrophysiological signals 102a-f as an electrophysiological measurement set 120. The electrophysiological signal standardizer 127 then calibrates the electrophysiological measurement set 120 based on the electrode position data set 118 to generate a calibrated electrophysiological measurement set 122.

In further examples, collecting the electrophysiological signals 102a-f and electrode position data 106a-f over the calibration time period 124 may be used to enable prediction models for certain pregnancy complications. In such an example, maternal hypertensive disorders may be detected based on pulse arrival times captured by measurement electrodes 200a-f of known position.

Fig. 10 schematically illustrates the controller 100 previously depicted in Figs. 2 and 4. The controller 100 includes the processor 125, the memory 175, and the transceiver 185. The memory 175 is configured to store the electrophysiological signals 102, the features 104 indicative of position of the measurement electrodes 200, the electrode position data 106, the user feedback data 108, the fetal heart rate data 114, the uterine activity data 116, the electrode position data set 118 collected over time, the electrophysiology measurement set 120 collected over time, the calibrated electrophysiology measurement set 122, and the calibration time period 124. The features 104 may include ECG waveform data 110, such as R-peak data 112. The processor 125 is configured to execute the electrophysiology signal generator 111, the feature extractor 113, the electrode position generator 115, the user feedback generator 117, the fetal heart rate data generator 119, the uterine activity generator 121, the electrophysiology signal collector 123, the electrode position data collector 125, and the signal standardizer 127.

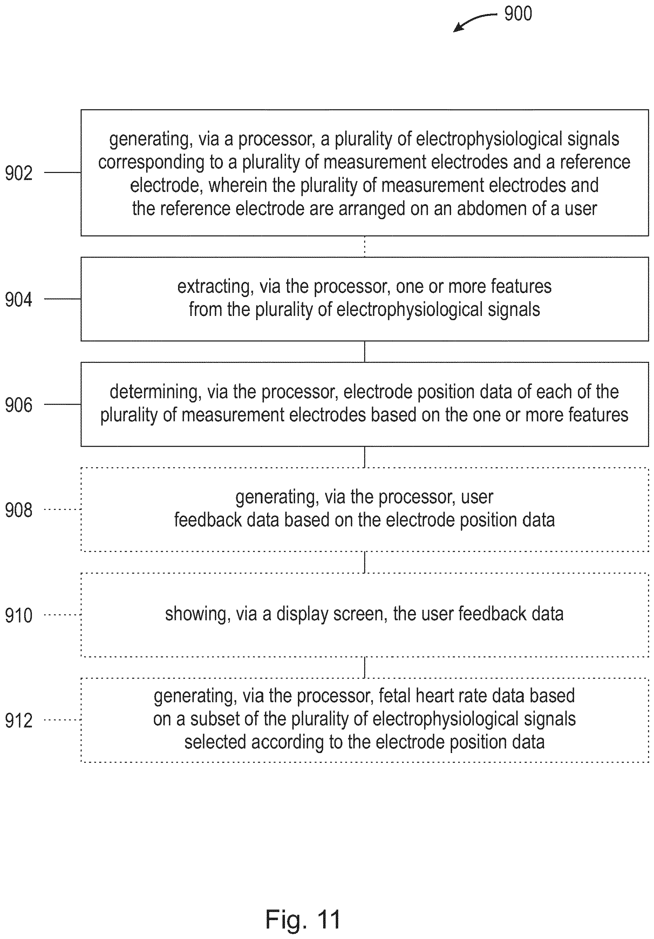

Fig. 11 is a flow chart of a method 900 for fetal monitoring. Referring to Figs. 1-11, the method 900 includes, in step 902, generating, via a processor 125, a plurality of electrophysiological signals 102 corresponding to a plurality of measurement electrodes 200 and a reference electrode 202. The plurality of measurement electrodes 200 and the reference electrode 202 are arranged on an abdomen AB of a user U.

The method 900 further includes, in step 904, extracting, via the processor 125, one or more features 104 from the plurality of electrophysiological signals 102.

The method 900 further includes, in step 906, determining, via the processor 125, electrode position data 106 of each of the plurality of measurement electrodes 200 based on the one or more features 104.

According to an example, the method 900 further includes, in step 908, generating, via the processor 125, user feedback data 108 based on the electrode position data 106. The method 900 further includes, in step 910, showing, via a display screen 500, the user feedback data 108.

According to an example, the method 900 further includes, in step 912, generating, via the processor 125, fetal heart rate data 114 based on a subset of the plurality of electrophysiological signals 102 selected according to the electrode position data 106.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

The above-described examples of the described subject matter can be implemented in any of numerous ways. For example, some aspects may be implemented using hardware, software, or a combination thereof. When any aspect is implemented at least in part in software, the software code can be executed on any suitable processor or collection of processors, whether provided in a single device or computer or distributed among multiple devices/computers.

The present disclosure may be implemented as a system, a method, and/or a computer program product at any possible technical detail level of integration. The computer program product may include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure may be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, including an object oriented programming language such as Smalltalk, C++, or the like, and procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some examples, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer readable program instructions.

The computer readable program instructions may be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various examples of the present disclosure. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Other implementations are within the scope of the following claims and other claims to which the applicant may be entitled.

While various examples have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the examples described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings is/are used. Those skilled in the art will recognize, or be able to ascertain using no more than routine experimentation, many equivalents to the specific examples described herein. It is, therefore, to be understood that the foregoing examples are presented by way of example only and that, within the scope of the appended claims and equivalents thereto, examples may be practiced otherwise than as specifically described and claimed. Examples of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present disclosure.

## Claims

1. A fetal monitoring system (10), comprising:
a plurality of measurement electrodes (200) configured to be arranged on an abdomen (AB) of a user (U);
a reference electrode (202) configured to be arranged on the abdomen (AB) of the user (U);
a processor (125) electrically coupled to the plurality of measurement electrodes (200) and the reference electrode (202), wherein the processor (125) is configured to:
generate a plurality of electrophysiological signals (102) corresponding to the plurality of measurement electrodes (200) and the reference electrode (202);
extract one or more features (104) from the plurality of electrophysiological signals (102);
determine electrode position data (106) of each of the plurality of measurement electrodes (200) based on the one or more features (104).

2. The fetal monitoring system (10) of claim 1, wherein the processor (125) is further configured to generate user feedback data (108) based on the electrode position data (106).

3. The fetal monitoring system (10) of claim 2, further comprising a display screen (500) configured to show the user feedback data (108).

4. The fetal monitoring system (10) of claim 2, further comprising a transceiver (185) configured to transmit the user feedback data (108) to an external device (600).

5. The fetal monitoring system (10) of claim 1, wherein the one or more features (104) include electrocardiogram (ECG) waveform data (110).

6. The fetal monitoring system (10) of claim 5, wherein the ECG waveform data (112) at least partially corresponds to maternal heart activity of the user (U).

7. The fetal monitoring system (10) of claim 5, wherein the electrode position data (106) is determined based on R-peak data (112) of the ECG waveform data (110).

8. The fetal monitoring system (10) of claim 1, wherein the processor (125) is further configured to generate fetal heart rate data (114) based on a subset of the plurality of electrophysiological signals (102) selected according to the electrode position data (106).

9. The fetal monitoring system (10) of claim 1, wherein the processor (125) is further configured to generate uterine activity data (116) based on one or more of the plurality of electrophysiological signals (102) and the electrode position data (106) of one or more of the plurality of measurement electrodes (200).

10. The fetal monitoring system (10) of claim 1, wherein the processor (125) is further configured to collect an electrode position data set (118) of the electrode position data (106) determined over a time period.

11. The fetal monitoring system (10) of claim 10, wherein the processor (125) is further configured to calibrate an electrophysiological measurement set (120) captured over the time period based on the electrode position data set (118).

12. The fetal monitoring system (10) of claim 1, further comprising at least one ground electrode (208).

13. A method (900) for fetal monitoring, comprising:
generating (902), via a processor, a plurality of electrophysiological signals corresponding to a plurality of measurement electrodes and a reference electrode, wherein the plurality of measurement electrodes and the reference electrode are arranged on an abdomen of a user;
extracting (904), via the processor, one or more features from the plurality of electrophysiological signals;
determining (906), via the processor, electrode position data of each of the plurality of measurement electrodes based on the one or more features.

14. The method (900) for fetal monitoring of claim 13, further comprising:
generating (908), via the processor, user feedback data based on the electrode position data; and
showing (910), via a display screen, the user feedback data.

15. The method (900) of fetal monitoring of claim 13, further comprising generating (912), via the processor, fetal heart rate data based on a subset of the plurality of electrophysiological signals selected according to the electrode position data.
